# EUROPÄISCHE PATENTANMELDUNG

(11) **EP 2 098 217 A2**
(43) Veröffentlichungstag der Anmeldung: **09.09.2009**
(21) Anmeldenummer: 09152047.8
(22) Anmeldetag: 04.02.2009
(51) Int. Cl.: A61K 8/04, A61Q 5/06, A61K 8/81, A61K 8/41, A61K 8/39, A61K 8/86

(54) **Volumengebender Haarfestiger mit ampholytischem Copolymer**

(30) Priorität: 07.03.2008 DE 102008013171
(71) Anmelder: Beiersdorf AG, 20253 Hamburg (DE)
(72) Erfinder: Krieger, Wiebke, 20259, Hamburg (DE); Franck, Kerstin, 25451, Quickborn (DE); Heuer, Björn, 22145, Hamburg (DE); Mahadeshwar, Anand, 22529, Hamburg (DE)

(57) **Zusammenfassung**

Kosmetische Zubereitung zur Behandlung keratinischer Strukturen, insbesondere menschlicher Haare enthaltend
a) ein Polymer A, aufgebaut aus den Monomeren Vinylpyrrolidon, *tert*-Butylacrylat, Methacrylsäure, Dimethylaminopropylmethacrylamid und Methacryloyloxyethyldimethylethyl Ammonium Ethylsulfat, wobei der Anteil Methacrylsäure geringer ist als der Anteil Dimethylaminopropylmethacrylamid und das Polymer mit einer geeigneten Säure neutralisiert ist,
b) ein kationisches Tensid K und/oder kationisches Polymer B,
c) ein nichtionisches Tensid N mit Polyoxyethylen- und/oder Polyoxypropyleneinheiten.

## Beschreibung

Die Erfindung betrifft neue Haarfestiger.

Zur temporären Verformung und Stabilisierung der Frisur werden Produkte eingesetzt, die als Stylingmittel bekannt sind. Hierzu zählen Haarsprays, Schaumfestiger, Gele, Wachse u.s.w.

Die Rezepturen sind vielfältig, die Bestandteile müssen in ethanolischem, wässrigethanolischem oder wässrigem Medium verträglich sein.

Üblicherweise bestehen diese Mittel zur Festigung der Haare aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation einen transparenten farblosen Film. Es werden eine Reihe von Anforderungen an diese Filme gestellt: sie sollen klar, glänzend, feuchtigkeitsunempfindlich, festigend, lang anhaltend, flexibel, nicht klebend sein, den natürlichen Griff des Haares nicht beeinträchtigen und sich auch wieder leicht durch handelsübliche Haarwaschmittel auswaschen lassen. Haarfestiger besitzen in der Gruppe der Stylingprodukte eine Sonderstellung. Nicht nur die Filmeigenschaften (Halt, Flexibilität...) stehen im Vordergrund, sondern auch die Pflegeleistung am Haar (Kämmbarkeit, gepflegter Griff). Haarfestiger werden von den meisten Konsumenten für eine Erhöhung des Haarvolumens bei der Frisurenerstellung verwendet.

Bei Schaumfestigern hat außerdem die Schaumcharakteristik (feinblasig, cremig, stabil) einen beträchtlichen Einfluss auf die Produktperformance.

Üblicherweise enthalten Stylingmittel weitere kosmetische Hilfsstoffe mit unterschiedlichen Funktionen, z.B. Pflegestoffe, Konservierungsmittel, Bakterizide, Parfüme, Emulgatoren, Treibmittel, weichmachende, anfeuchtende und/oder feuchthaltende Substanzen, Fette, Öle, Wachse, Fettalkohole, Fettsäureester oder andere übliche Bestandteile einer kosmetischen oder dermatologischen Formulierung wie Alkohole, Polyole, Polymere, Schaumstabilisatoren, Elektrolyte, organische Lösungsmittel, Silikone oder Siliconderivate, UV-Filter, Antioxidationsmittel, Extrakte, Proteine und/oder Proteinhydrolysate oder Pigmente enthalten.

Als haltgebende Polymere in Haarfestigern finden hauptsächlich statistische Copolymere Verwendung. Copolymerisiert werden in der Regel verschiedene nichtionische, anionische oder kationische / kationogene Monomeren mit Vinyl- oder Acryl-Funktion, wie z.B. Vinyllactame (Vinylpyrrolidon, Vinylcaprolactam), Vinylacetat, Alkyl(meth)acrylate (Methyl(meth)acrylat, Ethyl(meth)acrylat, Propyl(meth)acrylat, n-Butyl(meth)acrylat, tert-Butyl(meth)acrylat, Cyclohexyl(meth)acrylat, usw.), Styrol, (Meth)Acrylamid, Alkyl(meth)acrylamide, funktionalisierte Alkyl(meth)acrylate (z.B. Hydroxyethyl(meth)acrylat, Dimethylaminoethyl(meth)acrylat), Dimethylaminopropyl(meth)acrylat), funktionalisierte Alkyl(meth)acrylamide (Dimethylaminopropyl(meth)acrylamid), (Meth)Acrylsäure, Maleinsäure, Crotonsäure, Acrylamidomethylpropansulfonsäure, aminogruppenhaltige Monomere, deren Aminogruppe alkyliert (quarternisiert) vorliegt, wie (Meth)Acryloylethyltrialkyl Ammoniumchlorid, (Meth)Acryloylpropyltrialkyl Ammoniumchlorid, (Meth)Acryloylaminopropyltrialkyl Ammoniumchlorid, (Meth)Acryloylethyltrialkyl Ammoniummethosulfat, (Meth)Acryloylpropyltrialkyl Ammoniummethosulfat, (Meth)Acryloylaminopropyltrialkyl Ammoniummethosulfat. Beispiele für in Haarfestigern verwendete Polymere dieser Art sind z.B. Polymere mit der INCI Acrylates Copolymer (Luvimer®, Luviflex® Soft von BASF, Balance CR®, Balance O/55® von National Starch, Avalure AC® von Lubrizol), Acrylates/Hydroxyesters Acrylates Copolymer (Acudyne® von Rohm & Haas), Acrylates/Octylacrylamide Copolymer (Amphomer® oder Resyn XP von National Starch), AMP-Acrylates/Allyl Methacrylates Copolymer (Fixate® von Lubrizol), Butyl Ester of PVM/MA Copolymer (Gantrez® von ISP), Ethyl Ester of PVM/MA Copoylmer (Gantrez®, Omnirez® oder Advantage® von ISP), Acrylamide/Sodium Acryloyldimethyltaurate/Acrylic Acid Polymer (Acudyne SCP® von Rohm & Haas), Isobutylene/Ethylmaleimide/Hydroxyethylmaleimide Copolymer (Aquaflex FX-64® von ISP), Octylacrylamide/Acrylates/Butylaminoethyl Methacrylate Copolymer (Amphomer® oder Balance® von Natinal Starch), Acrylates/t-Butylacrylamid Copolymer (Ultrahold® von BASF), PEG/PPG-25/25 Dimethicone/Acrylates/t-Butylacrylates Copolymer (Luviflex Silk® von BASF), Polyvinylcaprolactam (Luviskol Plus® von BASF), PVM/MA Copolymer (Luviform FA® von BASF), PVP (Luviskol® von BASF), PVP/VA Copolymer (Luviskol® von BASF), VP/Dimethylaminoethylmethacrylate Copolymer (Copolymer® von ISP), Sodium Polystyrene Sulfonate (Flexan® von National Starch), VA/Crotonates/Vinyl Neodecanoate Copoylmer (Luviset CAN® von BASF, Resyn® von Natinal Starch), VA/Crotonates/Vinyl Propionate Copolymer (Luviset CAP® von BASF), VA/Butyl Maleate/Isobornyl Acrylate Copolymer (Advantage Plus® von ISP), VA/Crotonates Copolymer (Luviset CA 66® von BASF, Aristoflex® von Clariant), VA/Vinylbutylbenzoate/Crotonates Copolymer (Mexomere PW® von Chimex), Vinylcaprolactam/PVP/Dimethylaminoethyl Methacrylate Copolymer (Advantage® von ISP), VA/Butyl Maleate/Isobornyl Acrylate Copolymer (Advantage Plus® von ISP), Acrylates/C1-2 Succinates/HydroxyAcrylates Copolymer (Allianz LT-120® von ISP), VP/Vinyl Caprolactam/DMAPA Acrylates Copolymer (Aquaflex SF-40® von ISP), Vinyl Caprolactam/VP/Dimethylaminoethyl Methacrylate Copolymer (Gaffix® von ISP), VP/DMAPA Acrylates Copolymer (Styleze CC-1 0 von ISP), VP/Acrylates/Lauryl Methacrylate Copolymer (Styleze 2000® von ISP), VP/Methacrylamide/N-Vinyl Imidazole Copolymer (Luviset Clear® von BASF), alle Polyacrylat-Typen (Fixate Plus® und Fixate Superhold von Lubrizol, Luviset Shape® von BASF) und viele mehr.

Weitere Polymerklassen, die in Haarfestigern Einsatz finden sind z.B. Polyurethane, z.B. Polyurethane-1 (Luviset PUR® von BASF), Polyurethane-2 (Avalure UR® von Lubrizol), Polyurethane-6 (Luviset SI PUR® von BASF), Polyester, z.B. Polyester-5 (AQ-Polymere von Eastman), Polyimide, z.B. Polyimid-1 (Aquaflex XL-30® von ISP), und viele mehr.

Ein entscheidender Nachteil der haltgebenden Polymere in Haarfestigern sind die unzureichenden Pflegeeigenschaften: Das Haar lässt sich schlecht kämmen und fühlt sich stumpf, rau und wenig gepflegt an. Nach dem Trocknen sind die Filme dann relativ hart, spröde und unflexibel. Nachteilig für den Verbraucher sind die taktilen Eigenschaften des Filmes sowie die Bildung von Rückständen beim Kämmen (Filmplaken). Diese rieseln auf Schultern oder Kleidung und lassen den Anwender ungepflegt erscheinen.

Eine Verbesserung in Bezug auf Griff und Kämmbarkeit wird bei Haarfestiger-Formulierungen typischerweise durch die Kombination der genannten Polymere mit kationischen Polymeren, kationischen Tensiden oder Silikonen (beispielsweise cyclische Polydimethylsiloxane (Cyclomethicone), Silikontenside (Polyethermodifizierte Siloxane) vom Typ Dimethicone) erreicht.

Geeignete kationische Tenside sind ausgewählt aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide.

Geeignete kationische Polymere sind unter den Bezeichnungen Polyquaternium beschriebenen Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/- Dimethylaminoethylmethacrylat Copolymer (Polyquaternium-11), Homo- und Copolymere von Dimethyldiallylammoniumchlorid (Polyquaternium-6, -7 oder-22), quaternisierte Cellulosepolymere (Polyquaternium-4, -10), Copolymere aus kationischer Cellulose und Stärke (Polyquaternium-4 / Hydroxypropyl Starch Copolymer), quaternisiertes Vinylpyrrolidon / Dimethylaminopropylmethacrylat / Lauryl Dimethylaminopropylmethacrylat Copolymer (Polyquaternium-55), Vinylpyrrolidon/ Vinylimidazol/Methacrylamid/quaternisiertes Vinylimidazol (Polyquaternium-68), quaternisiertes Vinylcaprolactam/Vinylpyrrolidone/Dimethylaminopropyl methacrylamide /Methacryloylaminopropyllauryldimoniumchloride (Polyquaternium-69) oder quaternisierte Guarderivate.

Der Einsatz dieser kationischen Polymere oder kationischen Tenside führt jedoch meistens zu einer deutliche Abnahme der Volumeneigenschaften.

Außerdem erhält der Haarfestiger, falls dieser in Schaumform appliziert wird, durch den Einsatz vieler haltgebenden Filmbildner eine unzureichende, unkosmetische Schaumcharakteristik (grobblasig, weich, wenig cremig).

Zur Verbesserung der Schaumeigenschaften ist der Zusatz von schaumstabilsierenden Additiven, wie z.B. nichtionischen Tensiden üblich.

Geeignete nicht-ionische Tenside sind Alkohole, Alkanolamide, wie Cocamide MEA/ DEA/ MIPA, Aminoxide, wie Cocoamidopropylaminoxid,_Ester, die durch Veresterung von Carbonsäuren mit Ethylenoxid, Glycerin, Sorbitan oder anderen Alkoholen entstehen, Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid, Sucroseester, -Ether, Polyglycerinester, Diglycerinester, Monoglycerinester, Methylglucosester, sowie Ester von Hydroxysäuren.

Geeignete Treibmittel sind Kohlenwasserstoffe wie Butan, Isobutan und Propan, Dimethylether oder Fluorkohlenwasserstoffe wie z.B. 1,2-Diflourethan. Andere Treibmittel sind Stickstoff, Kohlendioxid, Distickstoffoxid oder komprimierte Luft.

Überaschenderweise wurden gefunden, dass eine kosmetische Zubereitung zur Behandlung keratinischer Strukturen, insbesondere menschlicher Haare enthaltend
a) ein Polymer A, aufgebaut aus den Monomeren Vinylpyrrolidon, *tert*-Butylacrylat, Methacrylsäure, Dimethylaminopropylmethacrylamid und Methacryloyloxyethyldimethylethyl Ammonium Ethylsulfat, wobei der Anteil Methacrylsäure geringer ist als der Anteil Dimethylaminopropylmethacrylamid und das Polymer mit einer geeigneten Säure neutralisiert ist,
b) ein kationisches Tensid K und/oder kationisches Polymer B,
c) ein nichtionisches Tensid N mit Polyoxyethylen- und/oder Polyoxypropyleneinheiten den Nachteilen des Standes der Technik abhilft.

Insbesondere kommt es zu einem synergistischen Effekt in Bezug auf die Volumeneigenschaften.

Dabei ist es bevorzugt, wenn Polymer A in Konzentrationen von 0,01 bis 30 Gew.-%, besonders bevorzugt 0,05 bis 20 Gew.-% , besonders bevorzugt 0,5 bis 15 Gew.-% enthalten ist. Dabei ist es bevorzugt, wenn Polymer A ein statistisches und lineares Copolymer ist. Dabei ist es bevorzugt, wenn Polymer A mit Phosphorsäure auf pH 6,0 neutralisiert wird. Dabei ist es bevorzugt, wenn die Konzentration an kationischem Tensid K und/oder kationischem Polymer B 0,01 bis 20 Gew.-% beträgt, bevorzugt 0,05 bis 3 Gew.-%, besonders bevorzugt 0,1 bis 2 Gew.-% Cetrimoniumchloride (Dehyquart A-CA® von Cognis oder CTAC 6025 KC® von Kci) und/oder 0,05 bis 3 Gew.-%, besonders bevorzugt 0,1 bis 2 Gew.-% Hydroxyethyl Cetyldimonium Phosphate (Luviquat Mono CP® von BASF) und/oder 0,5 bis 15 Gew.-%, besonders bevorzugt 1,0 bis 10 Gew.-% Polyquaternium-68 (Luviquat Supreme® von BASF) oder Mischungen davon. Dabei ist es bevorzugt, wenn das Tensid N in Konzentrationen von 0,01 bis 5 Gew.-% enthalten ist. Dabei ist es bevorzugt, wenn als Tensid N 0 bis 3 Gew.-%, besonders bevorzugt 0 bis 2 Gew.-% Laureth-3 (Dehydol LS3 Deo N® von Cognis) und/oder 0 bis 3 Gew.-%, besonders bevorzugt 0 bis 2 Gew.-% Trideceth-12 (Lipocol TD-12® von Lipo) enthalten sind, wobei die Gesamtkonzentration der Tenside N oder des Tensids N größer 0 ist. Dabei ist es bevorzugt, wenn 0 bis 25 Gew.-%, besonders bevorzugt 1 bis 25 Gew.-% Ethanol enthalten sind. Dabei ist es bevorzugt, wenn 0 bis 25 Gew.-%, besonders bevorzugt 1 bis 25 Gew.-% Treibmittel enthalten sind. Die Erfindung umfasst auch die Verwendung einer Zubereitung wie oben beschrieben als Haarfestiger, bevorzugt als treibmittelhaltiger Schaumfestiger, Pumpschaumfestiger oder als Pumpspray zur Applikation auf der keratinischen Substanz, insbesondere auf dem menschlichen Haar.

Erfindungsgemäße Haarfestiger können zusätzlich Pflegestoffe, Konservierungsmittel, Bakterizide, Parfüme, Emulgatoren, Treibmittel, weichmachende, anfeuchtende und/oder feuchthaltende Substanzen, Fette, Öle, Wachse, Fettalkohole, Fettsäureester oder andere übliche Bestandteile einer kosmetischen oder dermatologischen Formulierung wie Alkohole, Polyole, Polymere, Schaumstabilisatoren, Elektrolyte, organische Lösungsmittel, Silikone oder Siliconderivate, UV-Filter, Antioxidationsmittel, Extrakte, Proteine und/oder Proteinhydrolysate oder Pigmente enthalten.

Erfindungsgemäße Haarfestiger können zusätzlich mindestens ein weiteres anionisches, kationisches, nichtionisches oder amphoteres filmbildendes Polymer in einer Konzentration von 0 bis 10 Gew.-% enthalten.

### Beispiele

### Polymer 1:

statistisches, lineares Copolymer aus Vinylpyrrolidon, tert-Butylacrylat, Methacrylsäure, Dimethylaminopropylmethacrylamid und Methacryloyloxyethyldimethylethyl Ammonium Ethylsulfat, neutralisiert mit Phosphorsäure auf pH 6,0.

Polymer 1 ist erhältlich aus folgenden Monomeranteilen :
47 Gew.-% tert-Butylacrylat,
25 Gew.-% Vinylpyrrolidon,
10 Gew.-% Dimethylaminopropylmethacrylamid,
15 Gew.-% Methacryloyloxyethyldimethylethyl Ammonium Ethylsulfat,
3 Gew.-% Methacrylsäure.

Eine Untersuchung des Haarvolumens verschiedener Haarfestiger im Vergleich zu einer 3%igen Lösung von Polymer 1 ergab folgendes:

Zusatz von kationischem Tensid K und/oder kationischem Polymer B führte zu einer Volumenvergrößerung von 2 %. Zusatz von nichtionischem Tensid N mit Polyoxyethylen- und/oder Polyoxypropyleneinheiten führte zu einer Volumenvergrößerung von 4 %. Zusatz von kationischem Tensid K und/oder kationischem Polymer B und nichtionischem Tensid N mit Polyoxyethylen- und/oder Polyoxypropyleneinheiten führte zu einer Volumenvergrößerung von 20%.

### Rezepturbeispiele Haarfestiger:

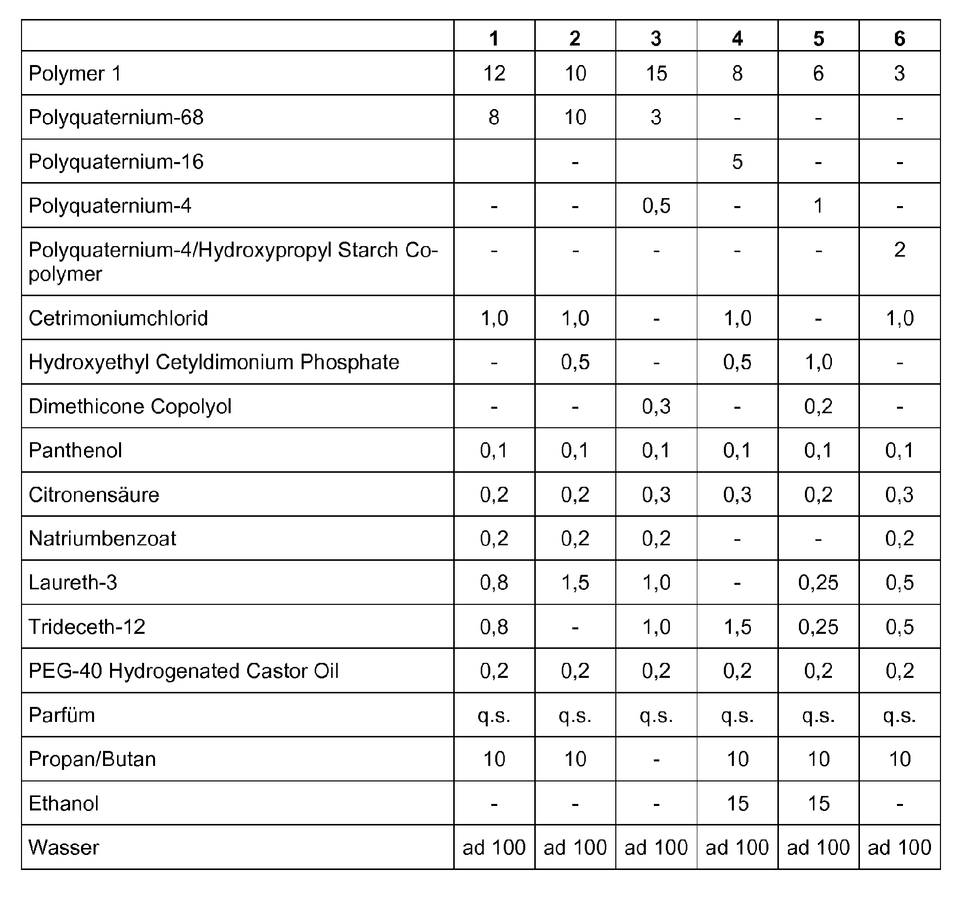

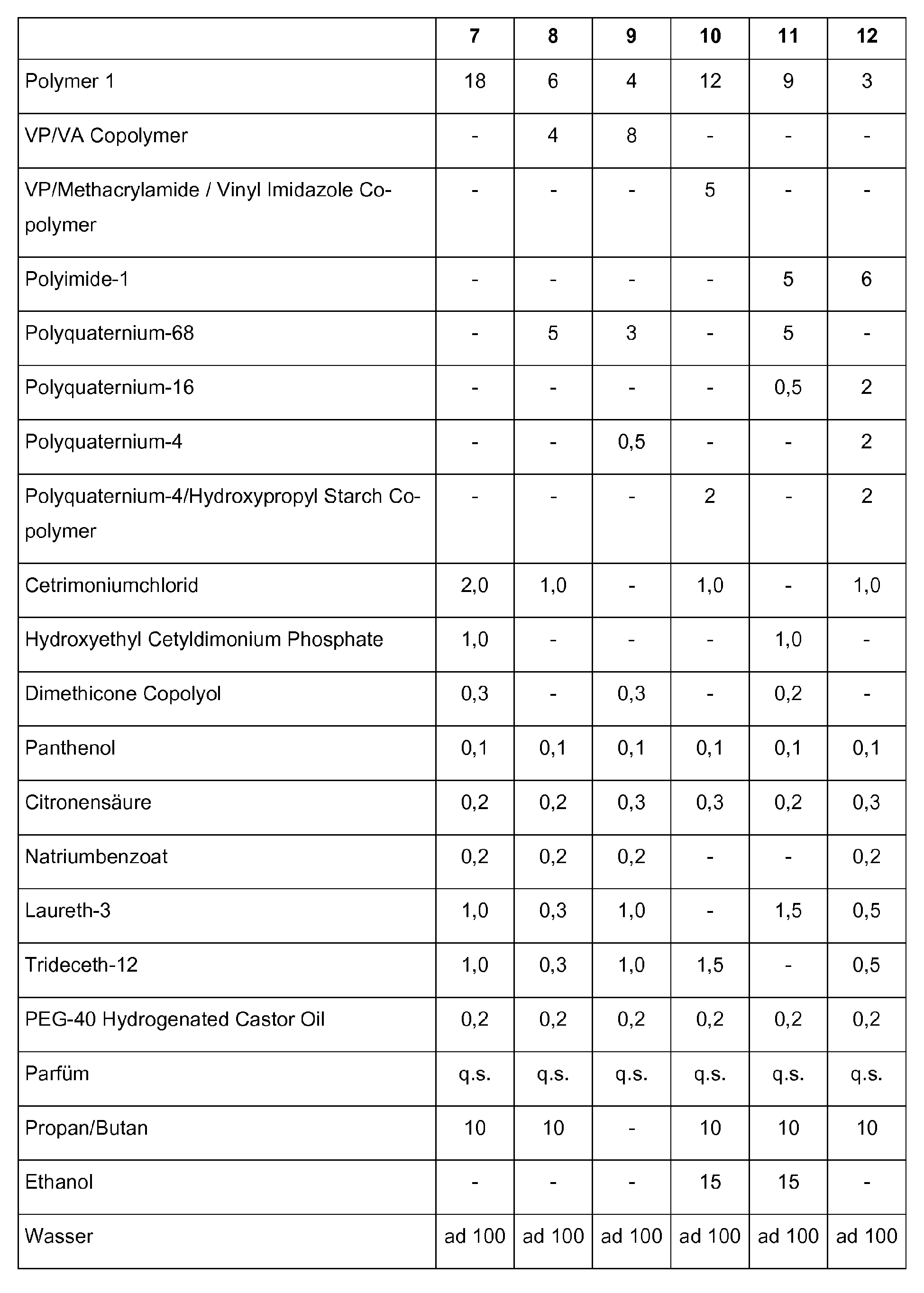

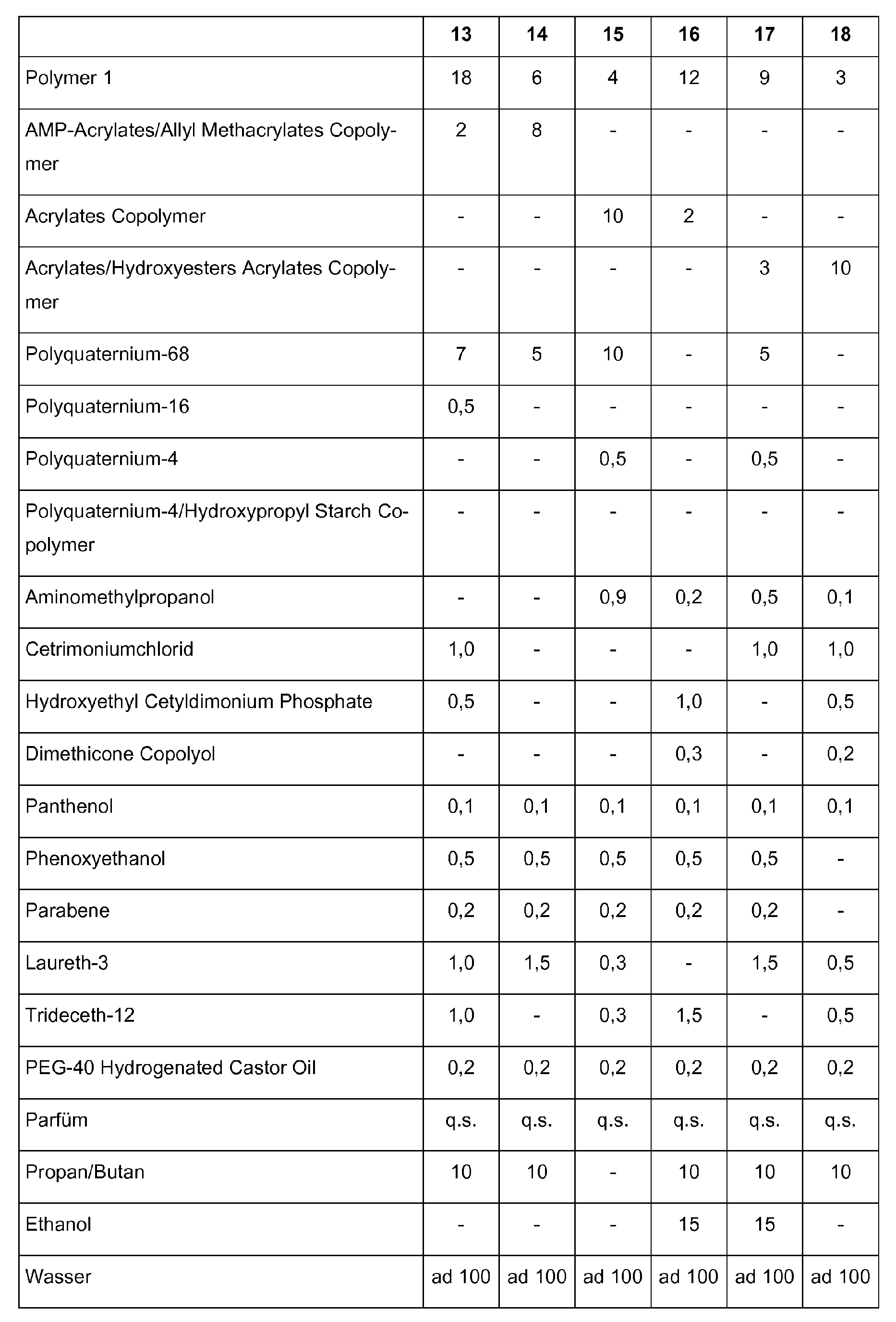

## Patentansprüche

1. Kosmetische Zubereitung zur Behandlung keratinischer Strukturen, insbesondere menschlicher Haare enthaltend
a) ein Polymer A, aufgebaut aus den Monomeren Vinylpyrrolidon, *tert*-Butylacrylat, Methacrylsäure, Dimethylaminopropylmethacrylamid und Methacryloyloxyethyldimethylethyl Ammonium Ethylsulfat, wobei der Anteil Methacrylsäure geringer ist als der Anteil Dimethylaminopropylmethacrylamid und das Polymer mit einer geeigneten Säure neutralisiert ist,
b) ein kationisches Tensid K und/oder kationisches Polymer B,
c) ein nichtionisches Tensid N mit Polyoxyethylen- und/oder Polyoxypropyleneinheiten.

2. Zubereitung nach Patentanspruch 1 **dadurch gekennzeichnet, dass** Polymer A in Konzentrationen von 0,01 bis 30 Gew.-%, besonders bevorzugt 0,05 bis 20 Gew.-% , besonders bevorzugt 0,5 bis 15 Gew.-% enthalten ist.

3. Zubereitungen nach einem der vorangehenden Patentansprüche **dadurch gekennzeichnet, dass** Polymer A ein statistisches und lineares Copolymer ist.

4. Zubereitungen nach einem der vorangehenden Patentansprüche **dadurch gekennzeichnet, dass** Polymer A mit Phosphorsäure auf pH 6,0 neutralisiert wird.

5. Zubereitungen nach einem der vorangehenden Patentansprüche **dadurch gekennzeichnet, dass** die Konzentration an kationischem Tensid K und/oder kationischem Polymer B 0,01 bis 20 Gew.-% beträgt, bevorzugt 0,05 bis 3 Gew.-%, besonders bevorzugt 0,1 bis 2 Gew.-% Cetrimoniumchloride (Dehyquart A-CA® von Cognis oder CTAC 6025 KC® von Kci) und/oder 0,05 bis 3 Gew.-%, besonders bevorzugt 0,1 bis 2 Gew.-% Hydroxyethyl Cetyldimonium Phosphate (Luviquat Mono CP® von BASF) und/oder 0,5 bis 15 Gew.-%, besonders bevorzugt 1,0 bis 10 Gew.-% Polyquaternium-68 (Luviquat Supreme® von BASF) oder Mischungen davon.

6. Zubereitungen nach einem der vorangehenden Patentansprüche **dadurch gekennzeichnet, dass** das Tensid N in Konzentrationen von 0,01 bis 5 Gew.-% enthalten ist.

7. Zubereitungen nach einem der vorangehenden Patentansprüche **dadurch gekennzeichnet, dass** als Tensid N 0 bis 3 Gew.-%, besonders bevorzugt 0 bis 2 Gew.-% Laureth-3 (Dehydol LS3 Deo N® von Cognis) und/oder 0 bis 3 Gew.-%, besonders bevorzugt 0 bis 2 Gew.-% Trideceth-12 (Lipocol TD-12® von Lipo) enthalten sind, wobei die Gesamtkonzentration der Tenside N oder des Tensids N größer 0 ist.

8. Zubereitungen nach einem der vorangehenden Patentansprüche **dadurch gekennzeichnet, dass** 0 bis 25 Gew.-%, besonders bevorzugt 1 bis 25 Gew.-% Ethanol enthalten sind.

9. Zubereitungen nach einem der vorangehenden Patentansprüche **dadurch gekennzeichnet, dass** 0 bis 25 Gew.-%, besonders bevorzugt 1 bis 25 Gew.-% Treibmittel enthalten sind.

10. Verwendung einer Zubereitung nach einem der vorangehenden Patentansprüche als Haarfestiger, bevorzugt als treibmittelhaltiger Schaumfestiger, Pumpschaumfestiger oder als Pumpspray zur Applikation auf der keratinischen Substanz, insbesondere auf dem menschlichen Haar.
